# EUROPEAN PATENT SPECIFICATION

(11) **EP 2 987 492 B1**
(45) Date of publication and mention of the grant of the patent: **01.05.2019**
(21) Application number: 13882519.5
(22) Date of filing: 18.04.2013
(51) Int. Cl.: A61K 31/565, A61P 19/02, A61K 9/00, A61K 9/107

(54) **USE OF 7-A-[9-(4,4,5,5,5 - PENTAFLUORO-PENTYL-SULFINYL)NONYL]-ESTRA-1,3,5(10)-TRIENE-3,17B-DIOL AND DERIVATIVES THEREOF**
VERWENDUNG VON 7A-[9-(4,4,5,5,5-PENTAFLUOR-PENTYL-SULFINYL-)NONYL-]ESTRA-1,3,5(10)-TRIEN-3,17B-DIOL UND DERIVATE DAVON
UTILISATION DE 7-A-[9-(4,4,5,5,5-PENTAFLUORO-PENTYL-SULFINYL)NONYL]-ESTRA-1,3,5(10)-TRIÈNE-3,17B-DIOL ET DE SES DÉRIVÉS

(43) Date of publication of application: 24.02.2016
(73) Proprietor: Xi'an Libang Pharmaceutical Technology Co., Ltd., Xi'an, Shaanxi 710065 (CN)
(72) Inventor: JIAO, Yaqi, Shaanxi 710077 Xi'an (CN); WANG, Jiucheng, Shaanxi 710077 Xi'an (CN); HU, Renle, Shaanxi 710077 Xi'an (CN)
(74) Representative: Lock, Graham James
(86) International application number: PCT/CN2013/074346
(87) International publication number: WO 2014/169456

(56) References cited:
- EP-A1- 2 987 799
- WO-A1-00/75660
- WO-A1-03/063859
- WO-A1-2011/014516
- WO-A2-2005/123193
- CN-A- 1 690 073
- DE-A1- 4 218 743
- FANG, ZHENYU ET AL.: 'Estrogens and Metabolites and Rheumatoid Arthritis' THE JOURNAL OF PRACTICAL MEDICINE vol. 28, no. 14, 2012, pages 2465 - 2467, XP008180866

## Description

### Technical Field

The invention belongs to the field of medicine, specifically relates to a novel medical use of a compound of general Formula A. Specifically, the invention relates to a novel use of 7-α-[9-(4,4,5,5,5-pentafluoropentylsulfinyl)nonyl]-estra-1,3,5(10)-triene-3,17β -diol and ester derivatives thereof in the treatment of TNF-related diseases such as rheumatism arthritis, rheumatoid arthritis and the like.

### Background Art

7-α-[9-(4,4,5,5,5-pentafluoropentylsulfinyl)nonyl]-estra-1,3,5(10)-triene-3,17p-diol, also referred to as fulvestrant, is a known estrogen receptor blocking agent generally used in the treatment of postmenopausal advanced breast cancer which fails to respond to anti-estrogen therapy and which is estrogen receptor positive.

Tumor necrosis factor -a (TNF-a) is the primary inflammatory cytokine in rheumatoid arthritis (RA), which is released by activated monocyte, macrophage and T lymphocyte. After binding to its receptor, TNF-α can function in immunoregulation and be involved in various pathophysiological processes of body, including fever, inflammation, infection, wound healing and tumor killing. Under normal circumstances, its expression and secretion are inhibited strictly by body, whereas it is expressed when involved in inflammation response in various diseases. It is involved in the regulation of inflammation which may lead to pathological changes in the joints, and its abnormal expression in RA is a key factor in the pathogenesis of RA. TNF-α is an important cytokine in inflammatory pathway, and is present in synovial tissues, synovial fluid and serum of patients with rheumatoid arthritis. TNF-α promotes RA inflammatory response, abnormal proliferation and apoptosis of synoviocytes, pannus formation and destruction of cartilage and bone through the interaction among TNF-α and various tissue factors and matrix proteins, and promotes continued progress of RA inflammatory response. Thus, TNF-α could be regarded as a clinical index of RA disease activity as well as an effective target for the treatment of RA.

Currently, the use of compounds of general Formula A, represented by fulvestrant, in the treatment of TNF-related diseases has not been reported.

WO 2011/014516 relates to estrogen antagonist as treatments for sclerosing disorders. The document discloses methods and compositions for treating a subject suffering from a sclerosing disorder, comprising administering, to the subject, an effective amount of an estrogen receptor antagonist (ERANT), wherein said ERANT has essentially no estrogen receptor agonist activity under physiological conditions.

WO 03/063859 relates to suppression of cartilage degradation via the estrogen receptor. The document discloses use of selective estrogen receptor modulators (SERMs) alone or in combination with progestins for treatment or prevention of diseases associated with elevated cartilage degradation.

EP 2987799 relates to an ester derivative of 7-alpha-[9-(4,4,5,5,5-pentafluoropentylsulphinyl)nonyl]oestra-1,3,5(10)-triene -3,17beta-diol having antitumour activity and preparation method thereof. The document discloses preparation of a class of fulvestrant ester derivatives.

CN 1690073 relates to steroid derivatives. The document discloses compounds for use in the treatment of mammary cancer.

### Summary of Invention

The object of the present invention is to provide a compound of Formula A for use in the treatment of arthritis.

The object of the present invention is achieved by the following technical solutions:
In one aspect, the present invention provides a compound of Formula A for use in the treatment of arthritis, 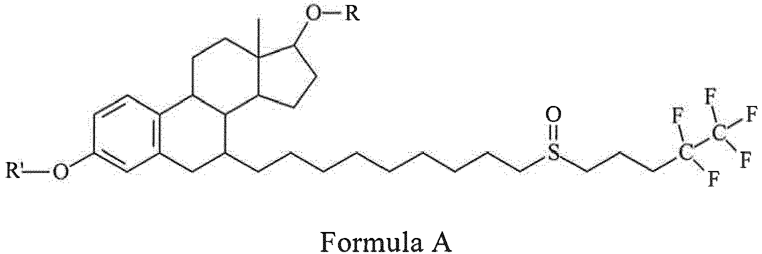 wherein,
substituent R' is selected from H, alkanoyl or alkenoyl having 2 to 4 carbon atoms;
substituent R is selected from H, alkanoyl or alkenoyl having 2 to 22 carbon atoms;
and when substituent R' is H, substituent R is linear or branched alkenoyl having 11 to 22 carbon atoms;
wherein, preferably, said arthritis is rheumatic arthritis or rheumatoid arthritis;
wherein, when R' is alkanoyl having 2 to 4 carbon atoms, said alkanoyl is acetyl;
preferably, said substituent R is alkenoyl having 11 to 22 carbon atoms;
preferably, substituent R contains one or more, preferably 1 to 6, carbon-carbon double bonds;
preferably, substituent R is branched alkenoyl, wherein said double bonds can be in the main chain or in the branched chain, preferably, substituent R is undec-2-enoyl;
preferably, said substituent R is alkanoyl having 11 to 22 carbon atoms;
preferably, said substituent R' is H, and substituent R is linear or branched alkanoyl having 11 to 22 carbon atoms;
preferably, substituent R is selected from undecanoyl, docosanoyl, octadecanoyl and isostearoyl.

Described herein is a method of treating a TNF-related disease, wherein, said method comprises administering to a subject in need a therapeutically effective amount of a compound of Formula A as described above;
preferably, said compound of Formula A is administered by injection;
preferably, said TNF-related disease is arthritis, more preferably, said arthritis is rheumatic arthritis or rheumatoid arthritis.

The present invention uses chicken type II collagen-induced arthritis model in SD rats to investigate the therapeutic effect of the compound of Formula A (specifically including the following Compounds I-VI) thereon.

After inflamed model was successfully established in SD rats, the SD rats were randomly divided into model control group (12 rats), low, medium dose group (8 rats for each), and high dose group (12 rats) of test drug depending on body weight. Rats in the model control group were administered a blank fat emulsion injection (0.5mL) by tail vein injection every other day for 7 days; rats in the low and medium dose group of test drug were administered a fat emulsion injection of a compound of Formula A (specifically including Compounds I-VI described hereafter) by tail vein injection every other day for 7 days; rats in the high dose group of test drug were administered a fat emulsion injection of a compound of Formula A by tail vein injection every other day for 14 days. 12 same models in the normal control group were also administered a blank fat emulsion injection. Indexes such as hindfoot joint score, hindfoot joint thickness measurement, TNF-α level in serum and hindfoot joint tissues, hindfoot joint pathological section and the like were used to evaluate efficacy of the drug.

The results showed that the compound of Formula A functions in lowering the swelling degree of hindfoot and the thickness of hindfoot ankle joint of rats, lowering the TNF-α level in serum, and lowering the TNF-α level in ankle joint significantly. Results of pathological section of the ankle joint of rats were substantially consistent with that of serology. This demonstrated that the compound of Formula A has anti-rheumatism arthritis and anti-rheumatoid arthritis effects.

### Description of the Drawings

Hereinafter, the embodiments of the present invention are illustrated in detail in combination with the drawings, wherein:
Figure 1 is a pathological section of hindfoot ankle joint of rats in the normal control group (×400);
Figure 2 is a pathological section of hindfoot ankle joint of rats in the model control group (×400);
Figure 3 is a pathological section of hindfoot ankle joint of rats in the high dose group of Compound I (1.40mg/kg) (×400);
Figure 4 is a pathological section of hindfoot ankle joint of rats in the high dose group of Compound V (1.80mg/kg) (×400);
Figure 5 is a pathological section of hindfoot ankle joint of rats in the high dose group of Compound II (1.80mg/kg) (×400);
Figure 6 is a pathological section of hindfoot ankle joint of rats in the high dose group of Compound III (2.16mg/kg) (×400);
Figure 7 is a pathological section of hindfoot ankle joint of rats in the high dose group of Compound IV (2.0mg/kg) (×400);
Figure 8 is a pathological section of hindfoot ankle joint of rats in the high dose group of Compound VI (1.48mg/kg) (×400).

### Description of the Preferred Embodiments

The present invention will be further described in combination with specific embodiments below. The examples provided are only for illustrating, but not for limiting the scope of the present invention.

Compound I used in the present invention was purchased from Xi'an Libang Pharmaceutical Co., Ltd.; Compounds II-IV were synthesized by the following method. SD rats used in the present invention were purchased from Super-B&K Laboratory Animal Corp. Ltd.; chicken type II collagen was purchased from Sigma, Lot No. 87H5227; rat tumor necrosis factor-α [TNF-α] enzyme-linked immunoassay kit (ELISA) was purchased from Shanghai Biovol Biotech Co., Ltd., Lot No. BV-E12102201.

The present invention uses type II collagen-induced arthritis model in SD rats to investigate the therapeutic effects of Compound I and its ester derivatives II-VI on arthritis (such as rheumatic arthritis and rheumatoid arthritis). Only examples covered by the scope of the claims form part of the invention.

### Example 1 Synthesis of Compound II

### 1) Reaction treatment

3g fulvestrant (4.95mmol) was added into a 250mL round-bottom flask, then dissolved with 160mL dichloromethane while stirring. Then, 0.0822g (0.66mmol) DMAP (4-dimethylaminopyridine), 0.96g (5.05mmol) undecanoic acid and1.02g (4.98mmol) DCC (N, N-dicyclohexylcarbodiimide) was added sequentially into said flask. After reacting under stirring at room temperature (e.g., 20±5°C) for 48h, the reaction was stopped.

### 2) Post process

The reaction system was first frozen to precipitate as much reaction by-product DCU (N,N'-dicyclohexylurea) as possible. After being filtered to remove solid DCU, the filtrate was washed with saturated sodium bicarbonate solution, then washed with water to neutral and evaporated by rotary evaporator to remove dichloromethane, to give colorless and clear colloidal liquid, which was dissolved in a small amount of ethyl acetate and then freezed in refrigerator (e.g., the freezing temperature may be -15±3°C) until no white solid DCU precipitated out. The filtrate was concentrated to remove ethyl acetate, recrystallized from mixed solvent of n-hexane-ethyl acetate, and filtered to remove white solid that precipitated out (unreacted raw material fulvestrant). The mother liquor was spin-dried to remove solvent and give colorless oily matter. Said oily matter was further purified by silica gel column chromatography (the eluent was n-hexane-ethyl acetate (1:1, volume ratio)), and was then evaporated by rotary evaporator to give 1.0611g of colorless oily matter, which was Compound II (purity 99.104% as determined by HPLC, C18 column, mobile phase: 67% THF(tetrahydrfuran) in water, flow rate: 1.0 mL/min, detection wavelength: 220nm)), and the molar yield was 27.7%.
IR(cm-1): 3385, 2926, 2855, 1756, 1494, 1463, 1199, 1152, 1059, 1017, 985, 721.
¹HNMR(500 MHz, CDC13, ppm): δ 7.28 (s, 1 H), 6.83 (d, 1 H), 6.77 (d, 1 H), 3.73 (t, 1 H, J=8 Hz), 2.88-1.17 (t, 57 H), 0.89 (s, 3 H), 0.77 (s, 3 H).
¹³CNMR(125 MHz, CDC13, ppm): δ 172.64, 148.52, 137.13, 126.91, 122.37, 120.10, 118.64, 81.93, 52.75, 51.03, 46.47, 43.33, 41.67, 38.23, 36.89, 34.50, 34.45, 33.85, 31.89, 29.67, 29.50, 29.63, 29.55, 29.49, 29.46, 29.34, 29.30, 29.26, 29.16, 29.12, 28.80, 28.23, 27.11, 25.70, 25.01, 24.88, 22.66, 14.62, 14.50, 11.50.

### Example 2 Synthesis of Compound III

### 1) Reaction treatment

3g fulvestrant (4.95mmol) was added into a 250mL round-bottom flask, then dissolved with 160mL dichloromethane while stirring. Then, 0.0822g (0.66mmol) DMAP, 1.87g (5.05mmol) docosanoic acid and 1.02g (4.98mmol) DCC was added sequentially into said flask. After reacting under stirring at room temperature (e.g., 20±5°C) for 48h, the reaction was stopped.

### 2) Post process

Reaction liquid was treated according to the post process in Example 1 to give 1.016g of white solid powder (purity 92.634% by HPLC) (C18 column, mobile phase: 75% THF in water, flow rate: 1.0 mL/min, detection wavelength: 220nm), which was Compound III, and the molar yield was 22.1%.
IR(cm-1): 3607, 3424, 2919, 2851, 1754, 1495, 1471, 1199, 1153, 1141, 1112, 1081, 985, 719.
¹HNMR(500 MHz, CDC13, ppm): δ 7.28 (d, 1 H), 6.83 (d, 1 H), 6.77 (d, 1 H), 3.74 (t, 1 H, J=8 Hz), 2.91-1.05 (t, 79 H), 0.89 (t, 3 H), 0.77 (s, 3 H).
¹³CNMR(125 MHz, CDC13, ppm): δ 172.64, 148.53, 137.13, 126.91, 122.37, 118.64, 81.93, 52.83, 51.11, 46.48, 43.34, 41.68, 38.24, 36.89, 34.50, 33.15, 31.94, 30.56, 29.94, 29.86, 29.71, 29.67, 29.63, 29.62, 29.51, 29.48, 29.37, 29.35, 29.27, 29.17, 29.13, 28.81, 28.23, 27.12, 25.70, 25.01, 24.88, 23.16, 22.66, 14.50, 14.01, 11.50.

### Example 3 Synthesis of Compound IV

3g fulvestrant (4.95mmol) was added into a 250mL round-bottom flask, then dissolved with 160mL dichloromethane while stirring. Then, 0.0822g (0.66mmol) DMAP, 1.44g (5.05mmol) isostearic acid and 1.02g (4.98mmol) DCC was added sequentially into said flask. After reacting under stirring at room temperature (e.g., 20±5°C) for 48h, the reaction was stopped.

Reaction liquid was treated according to the post process in Example 1 to give 1.0028g of colorless colloid (purity 99.312%, determined by HPLC) (with the same method in Example 2), which was Compound IV, and the molar yield was 23.2%.
IR(cm-1): 3396, 2928, 2866, 1748, 1494, 1466, 1364, 1198, 1149, 1121, 1058, 1017, 984, 720.
¹HNMR(500 MHz, CDC13, ppm): δ7.28 (s, 1 H), 6.83 (d, 1 H), 6.76 (s, 1 H), 3.74 (t, 1 H, J=8 Hz), 2.35-1.03 (t, 71 H), 1.09-0.94 (t, 3 H), 0.89 (s, 3 H), 0.77 (s, 3 H).
¹³CNMR (125 MHz, CDC13, ppm): δ 171.15, 148.60, 137.03, 126.88, 122.45, 118.72, 81.94, 53.34, 53.04, 52.82, 51.39, 50.96, 48.46, 48.39, 48.32, 46.48, 43.33, 41.68, 38.21, 37.92, 37.86, 37.79, 36.89, 34.50, 33.16, 32.37, 32.05, 31.11, 30.56, 30.06, 29.96, 29.87, 29.69, 29.55, 29.50, 29.37, 29.32, 29.18, 28.81, 28.26, 27.11, 26.09, 25.65, 24.81, 22.66, 21.21, 19.40, 14.61, 14.50, 11.51.

### Example 4 Synthesis of Compound V

0.36g fulvestrant (0.6mmol) was added into a 50mL round-bottom flask, then dissolved with 25 mL dichloromethane while stirring. Then, 9.93mg (0.08mmol) DMAP ,0.113g (0.61mmol) undecenoic acid and 0.13g (0.64 mmol) DCC was added sequentially into said flask. After reacting under stirring at room temperature (e.g. 20±5°C) for 48h, the reaction was stopped.

Reaction liquid was treated according to the post process in Example 2 to give light yellow oily matter. Said oily matter was further purified by silica gel column chromatography for 3 times and neutral alumina for once, and was evaporated to dryness to give 0.1g of light yellow oily matter (purity 96.010%, determined by HPLC) (with the same method in Example 2), which was Compound V, and the yield was 21.5%.
IR(KBr, cm-1): 3387, 2927, 2855, 1736, 1652, 1494, 1461, 1356, 1312, 1198, 1154, 1121, 1059, 1016, 983, 721.
¹HNMR(500 MHz, CDCl3, ppm): δ 7.27 (t, 1 H), 7.15 (t, 1 H), 6.87 (s, 1 H), 6.82 (s, 1 H), 6.43 (t, 2 H), 5.99 (t, 1 H), 3.74 (t, 1 H, J=8 Hz), 3.2-1.1 (t, 51 H), 0.89 (t, 3 H, J=7 Hz), 0.77 (s, 3 H).
¹³CNMR(125 MHz, CDCl3, ppm): δ 170.90, 165.38, 151.71, 148.55, 137.10, 135.55, 126.91, 122.44, 120.94, 120.12, 118.79, 81.93, 52.77, 51.04, 46.50, 43.35, 41.71, 38.27, 36.91, 34.51, 33.18, 31.85, 30.56, 29.94, 29.87, 29.70, 29.62, 29.51, 29.36, 29.34, 29.19, 29.16, 29.09, 28.96, 28.81, 28.23, 27.13, 25.70, 24.88, 22.66, 14.50, 13.50, 11.10.

### Example 5 Synthesis of Compound VI

0.31g (0.4mmol) Compound V (synthesized in Example 4) 4 mL (40 mmol) acetic anhydride, 0.2 g (1.6 mmol) 4-dimethylaminopyridine (DMAP) was sequentially added into a 50mL round-bottom flask. Then, 30 mL THF solution was added into said flask. After reflux reacting for 48 h, the reaction was stopped.

After the reaction system was cooled, it was washed with water to neutral and phase separated. The organic layer was spin-dried and purified by silica gel column chromatography through gradient eluting (the eluent was n-hexane-ethyl acetate (40: 1/10: 1/5: 1, volume ratio)). Then, the eluent was evaporated to dryness to give milky white colloidal liquid, which was Compound VI.
IR(cm-1): 3449, 2927, 2855, 1736, 1651, 1494, 1461, 1373, 1360, 1311, 1245, 1198, 1154, 1121, 1045, 1027, 983, 896, 822, 720.
¹HNMR(500 MHz, CDCl3, ppm): δ 7.27 (t, 1 H), 7.15(t, 1 H), 6.87(t, 1 H), 6.81 (d, 1 H), 6.40 (t, 1 H), 6.00 (d, 1 H), 5.63 (t, 1 H), 4.70 (t, 1 H), 2.7-1.1 (t, 52 H), 2.05 (t, 3 H), 0.89 (t, 3 H), 0.82 (s, 3 H).
¹³CNMR(125 MHz, CDC13, ppm): δ 170.90, 165.36, 151.72, 148.56, 137.07, 136.97, 126.92, 122.43, 122.32, 120.92, 118.73, 82.76, 52.71, 50.98, 46.26, 42.94, 41.40, 38.20, 38.12, 37.06, 34.50, 33.17, 32.50, 32.41, 31.84, 29.85, 29.67, 29.55, 29.49, 29.35, 29.32, 29.18, 29.15, 28.79, 28.16, 26.96, 25.64, 22.78, 22.65, 21.17, 14.63, 12.02.

### Example 6 Pharmacodynamic experiment

### Establishment of model:

Chicken type II collagen was dissolved in O.lmol/L acetic acid at 4 °C while stirring so as to be thoroughly dissolved at a concentration of 2mg/mL. The resulting solution was put into a refrigerator at 4 °C overnight. Liquid paraffin and lanolin were mixed at a ratio of 2:1 (volume ratio), and the mixture was autoclaved to prepare an incomplete Freund's adjuvant (IFA). Bacillus Calmette Guerin (BCG) vaccine (60mg) was inactivated at 80 °C for 1 hour and transferred to a mortar, into which was dropwise added IFA. After being uniformly grinded, the resulting mixture was pushed and sucked repeatedly by double-syringe to prepare a complete Freund's adjuvant (CFA), with the final concentration of BCG vaccine being 2mg/mL. An equal amount of chicken type II collagen acid solution was mixed with CFA into a stable emulsion (containing 1mg chicken type II collagen per milliliter).

12 female SD rats were randomly selected from 192 female SD rats as normal control group. The remaining 180 rats was disinfected with iodine tincture. After being deiodinated with 75% ethanol, each of the rat was administered 150µL collagen emulsion by intradermal injection at the base of the tail, and was administered 100µL collagen emulsion by intradermal injection at the vola pedis of right hindfoot. Thus, totally 250µL for each rat was administered. 7 days later, an equal amount of collagen emulsion was intraperitoneal injected as a secondary immune.

Animal grouping and administration: inflamed rats were divided into model control group, low, medium and high dose groups of test drug based on body weight. Administration was carried out according to the drug, group, dose and administration time as shown in the following Table 1:

**Table 1 Drug, group, dose and administration time**

| **Group** | | **Number of animal** | **Administration method** | **Administration dose** | **Administration time** |
|---|---|---|---|---|---|
| **Normal control group (blank fat emulsion)** | | 12 | tail vein injection | 0.5mL | every other day, for 7 days |
| **Model control group (blank fat emulsion)** | | 12 | tail vein injection | 0.5mL | every other day, for 7 days |
| **Compound I** | **Low dose group** | 8 | tail vein injection | 0.35 mg/kg | every other day, for 7 days |
| | **Medium dose group** | 8 | tail vein injection | 0.70 mg/kg | every other day, for 7 days |
| | **High dose group** | 12 | tail vein injection | 1.40 mg/kg | every other day, for 14 days |
| **Compound II** | **Low dose group** | 8 | tail vein injection | 0.45 mg/kg | every other day, for 7 days |
| | **Medium dose group** | 8 | tail vein injection | 0.9 mg/kg | every other day, for 7 days |
| | **High dose group** | 12 | tail vein injection | 1.8 mg/kg | every other day, for 14 days |
| **Compound III** | **Low dose group** | 8 | tail vein injection | 0.54 mg/kg | every other day, for 7 days |
| | **Medium dose group** | 8 | tail vein injection | 1.08 mg/kg | every other day, for 7 days |
| | **High dose group** | 12 | tail vein injection | 2.16 mg/kg | every other day, for 14 days |
| **Compound IV** | **Low dose group** | 8 | tail vein injection | 0.5 mg/kg | every other day, for 7 days |
| | **Medium dose group** | 8 | tail vein injection | 1.0 mg/kg | every other day, for 7 days |
| | **High dose group** | 12 | tail vein injection | 2.0 mg/kg | every other day, for 14 days |
| **Compound V** | **Low dose group** | 8 | tail vein injection | 0.45 mg/kg | every other day, for 7 days |
| | **Medium dose group** | 8 | tail vein injection | 0.9 mg/kg | every other day, for 7 days |
| | **High dose group** | 12 | tail vein injection | 1.8 mg/kg | every other day, for 14 days |
| **Compound VI** | **Low dose group** | 8 | tail vein injection | 0.37mg/kg | every other day, for 7 days |
| | **Medium dose group** | 8 | tail vein injection | 0.74mg/kg | every other day, for 7 days |
| | **High dose group** | 12 | tail vein injection | 1.48mg/kg | every other day, for 14 days |

| | | | | | |
|---|---|---|---|---|---|
| Note: each group began to be administered on the 20^{th} day after being inflamed; 8 rats were sacrificed on the 28^{th} day, the rest continued to be administered in accordance with the dosing interval; the rest of the animals were sacrificed on the 35^{th} day, and histologyl index detection was carried out. | | | | | |

wherein, test drugs are compound I-VI as shown below:
Compound I: 7-α-[9-(4,4,5,5,5-pentafluoropentylsulfinyl)nonyl]-estra-1,3,5(10)-triene-3,17β -diol
Compound II: 7-α-[9-(4,4,5,5,5-pentafluoropentylsulfinyl)nonyl]-estra-1,3,5(10)-triene-3-hyd roxyl-17-undecanoyl
Compound III: 7-α-[9-(4,4,5,5,5-pentafluoropentylsulfinyl)nonyl]-estra-1,3,5(10)-triene-3-hyd roxyl-17-docosanoyl
Compound IV: 7-α-[9-(4,4,5,5,5-pentafluoropentylsulfinyl)nonyl]-estra-1,3,5(10)-triene-3-hyd roxyl-17-isostearoyl
Compound V: 7-α-[9-(4,4,5,5,5-pentafluoropentylsulfinyl)nonyl]-estra-1,3,5(10)-triene-3-hyd roxyl-17-(undec-2-enoyl)
Compound VI: 7-α-[9-(4,4,5,5,5-pentafluoropentylsulfinyl)nonyl]-estra-1,3,5(10)-triene-3-ace tyl-17-(undec-2-enoyl

All of these test drugs were administered after fat emulsion injection.

### Evaluation of results and data collection

Rats in each group were scored for the hindfoot joint and measured for the thickness of ankle joint before being inflamed. Normal rats were taken blood from orbital venous plexus. The serum of the blood was separated out and cryopreserved at -20 °C to detect cytokines. Rats were scored for joint after being inflamed for 3 days, and were measured for the thickness of ankle joint before administration (on the 19^{th} day since being inflamed), on the 3^{rd} day and 8^{th} day after administration (on the 20^{th} day since being inflamed). Rats in each group were taken blood from orbital venous plexus when inflammation was severe (on the 23^{rd} day after being inflamed). The serum of the blood was separated out and cryopreserved at -20 °C. 8 rats for each group were sacrificed on the 28^{th} day after being inflamed and were taken blood from intraperitoneal vein. The serum of the blood was separated out and was subjected to serological detection (TNF-a). The rest of rats were sacrificed on the 35^{th} day after being inflamed, and histology index detection was carried out

### Test results

### (1) Hindfoot joint score results

Hindfoot joint score results of rats after the establishment of model and the administration were shown in Table 2. Compared with the normal control group, the joint score of the model control group was significantly increased (p <0.01). Inflammation was most severe on the 3^{rd} day after administration. The joint score of the model control group was the highest. The joint score of the administration groups decreased and most of which had significant differences compared with the model control group (p<0.01).

**Table 2 Hindfoot joint score results of arthritis model in rats**

| **Group** | | **n** | **Mean value of hindfoot joint score** | | |
|---|---|---|---|---|---|
| | | | **Before administration** | **The 3^{rd} Day after administration** | **The 8^{th} Day after administration** |
| **Normal control group** | | 12 | 0.8** | 1 | 1.1 |
| **Model control group** | | 12 | 5.4 | 6.2^{#} | 5.4 |
| **Compound I** | **Low dose group** | 8 | 4.8 | 4.8** | 5.9^{##} |
| | **Medium dose group** | 8 | 4.5 | 5.3**^{##} | 5.1* |
| | **High dose group** | 12 | 5.1 | 4.9** | 4.9^{#} |
| **Compound II** | **Low dose group** | 8 | 4.9 | 4.8** | 5.6^{#} |
| | **Medium dose group** | 8 | 4.9 | 4.9** | 4.9 |
| | **High dose group** | 12 | 4.7 | 5.3**^{#} | 4.9 |
| **Compound III** | **Low dose group** | 8 | 4.5 | 5.3**^{##} | 5 |
| | **Medium dose group** | 8 | 5 | 4.8** | 5.4 |
| | **High dose group** | 12 | 5.4 | 4.8**^{#} | 5.6 |
| **Compound IV** | **Low dose group** | 8 | 4.5 | 5.3*^{#} | 5.3^{#} |
| | **Medium dose group** | 8 | 4.4 | 4.8** | 5.6^{##} |
| | **High dose group** | 12 | 4.7 | 4.9** | 5.1 |
| **Compound V** | **Low dose group** | 8 | 4.6 | 5.1** | 5.9^{##} |
| | **Medium dose group** | 8 | 4.4 | 5.7^{##} | 4.4* |
| | **High dose group** | 12 | 4.5 | 5.6^{##} | 4.8 |
| **Compound VI** | **Low dose group** | 8 | 4.6 | 5.4**^{##} | 4.9 |
| | **Medium dose group** | 8 | 4.8 | 4.6** | 5.5 |
| | **High dose group** | 12 | 5.3 | 4.7**^{#} | 5.5 |

| | | | | | |
|---|---|---|---|---|---|
| Note: n denotes the number of animals (the same below); compared with the model control group, *p<0.05, **p<0.01; compared with itself before administration, ^{#}p<0.05, ^{##}p<0.01. | | | | | |

### (2) Determination of the thickness of hindfoot ankle joint

Table 3 showed the changes in the thickness of right ankle joint of rats with arthritis after administration. Determination results show that, compared with the normal control group, the thickness of ankle joint of the model control group significantly increased (p<0.01), while the thickness of ankle joint in each administration group decreased compared with that in the model control group, with high dose group having significant difference (p<0.01). The comparison of itself before and after administration showed that, the thickness of hindfoot ankle joint of rats in each group of test drug continued to increase at the early stage of administration, and decreased with the extension of administration time.

**Table 3. Measurement results of the thickness of right ankle joint of rat by microcalliper (mean±SD (standard deviation))**

| **Group** | | **n** | **Thickness of ankle joint (mm)** | | |
|---|---|---|---|---|---|
| | | | **Before administration** | **The 3^{rd} Day after administration** | **The 8^{th} Day after administration** |
| **Normal control group** | | 12 | 5.29±0.33** | 5.41±0.33** | 5.11±0.39**^{##} |
| **Model control group** | | 12 | 6.77±0.68 | 6.76±0.68 | 6.67±0.68 |
| **Compound I** | **Low dose group** | 8 | 6.39±0.44 | 6.15±0.54 | 6.20±0.52 |
| | **Medium dose group** | 8 | 6.14±0.35 | 6.22±0.46 | 6.05±0.31* |
| | **High dose group** | 12 | 6.25±0.47 | 6.04±0.51 | 5.98±0.21** |
| **Compound II** | **Low dose group** | 8 | 6.39±0.68 | 6.05±0.55 | 5.98±0.19* |
| | **Medium dose group** | 8 | 5.99±0.57 | 6.31±0.54 | 6.31±0.51 |
| | **High dose group** | 12 | 6.31±0.52 | 6.29±0.69 | 5.83±0.61** |
| **Compound III** | **Low dose group** | 8 | 5.94±0.52 | 6.22±0.38 | 6.06±0.29* |
| | **Medium dose group** | 8 | 6.33±0.52 | 6.45±0.53 | 6.36±0.46 |
| | **High dose group** | 12 | 6.59±0.44 | 6.55±0.56 | 6.31±0.69 |
| **Compound IV** | **Low dose group** | 8 | 6.10±0.39 | 6.32±0.41 | 6.41±0.47 |
| | **Medium dose group** | 8 | 6.14±0.59 | 6.48±0.48 | 6.22±0.42 |
| | **High dose group** | 12 | 6.09±0.57 | 6.08±0.51 | 5.98±0.39** |
| **Compound V** | **Low dose group** | 8 | 6.27±0.36 | 6.36±0.64 | 6.20±0.52 |
| | **Medium dose group** | 8 | 6.09±0.45 | 6.74±0.44^{#} | 6.15±0.37 |
| | **High dose group** | 12 | 6.28±0.44 | 6.44±0.60 | 6.07±0.41* |
| **Compound IV** | **Low dose group** | 8 | 6.13±0.30 | 6.43±0.21 | 6.33±0.57 |
| | **Medium dose group** | 8 | 6.34±0.51 | 6.58±0.28 | 6.42±0.52 |
| | **High dose group** | 12 | 5.98±0.77 | 6.02±0.46 | 5.69±0.40** |

| | | | | | |
|---|---|---|---|---|---|
| Note: compared with the model control group, *p<0.05, **p<0.01. | | | | | |

### (3) Determination results of TNF-α in serum

Determination results of TNF-α in serum of rats in each group were shown in table 4. On the 3^{rd} day after the rats were administered, the TNF-α level in serum in each group significantly increased compared with that in the normal group, with the most significant increase in the model control group (p<0.01), while the TNF-α level in the administration groups significantly decreased compared with the model control group, with the high dose group having significant difference (p<0.01). On the 8^{th} day after administration, the TNF-α level in serum in the model control group continued to increase, and the TNF-α level in the low dose group slightly increased, but still lower than that in the model control group, with the results having significant difference.

**Table 4. Effect on TNF-α level in serum of rats with arthritis (mean±SD)**

| **Group** | | **n** | **TNF-α level in serum (pg/mL)** | |
|---|---|---|---|---|
| | | | **The 3^{rd} day after administration** | **The 8^{th} Day after administration** |
| **Normal control group** | | 12 | 6.34±6.72 | 16.16±10.30** |
| **Model control group** | | 12 | 62.79±14.04 | 160.82±91.10 |
| **Compound I** | **Low dose group** | 8 | 50.15±60.06 | 52.78±67.16* |
| | **Medium dose group** | 8 | 31.55±54.58** | 22.41±25.25** |
| | **High dose group** | 12 | 13.48±25.05** | 18.45± 12.72** |
| **Compound II** | **Low dose group** | 8 | 48.28±94.84 | 201.35±250.23 |
| | **Medium dose group** | 8 | 11.00±9.39** | 75.12±93.40 |
| | **High dose group** | 12 | 26.72±35.26** | 25.65±20.66** |
| **Compound III** | **Low dose group** | 8 | 81.94±141.25 | 157.89±248.01 |
| | **Medium dose group** | 8 | 12.31±17.56** | 39.56±29.29** |
| | **High dose group** | 12 | 16.53±15.71** | 10.36±7.08** |
| **Compound IV** | **Low dose group** | 8 | 32.45±57.30 | 71.57±111.19 |
| | **Medium dose group** | 8 | 8.60±8.44** | 63.77±73.71* |
| | **High dose group** | 12 | 50.34±94.70 | 19.13±12.84** |
| **Compound V** | **Low dose group** | 8 | 66.71±121.06 | 123.73±157.16 |
| | **Medium dose group** | 8 | 10.02±14.59** | 23.44±27.21** |
| | **High dose group** | 12 | 46.48±101.05 | 12.35±9.72** |
| **Compound VI** | **Low dose group** | 8 | 47.22±96.55 | 197.35±231.02 |
| | **Medium dose group** | 8 | 13.08±7.93** | 78.21±88.90 |
| | **High dose group** | 12 | 28.27±37.62** | 26.56±18.06** |

| | | | | |
|---|---|---|---|---|
| Note: compared with the model control group, *p<0.05, **p<0.01 | | | | |

### (4) Determination results of TNF-α in hindfoot ankle joint tissue

Determination results of the TNF-α content in hindfoot ankle joint of rats in each group were shown in table 5. The TNF-α level in left and right hindfoot ankle joint in each administration group was significantly higher than that of rats in the normal group (p<0.01, p<0.05). In left ankle joint, the TNF-α level in ankle joint significantly decreased in each treatment group, with significant difference compared with the model control group (p<0.01), while had no obvious difference compared with the TNF-α content in ankle joint of rats in the normal group. In right ankle, the TNF-α level in ankle joint also significantly decreased in each treatment group, but the overall effect was not as good as that in left ankle.

**Table 5. Effect on TNF-α level in ankle joint of rats with arthritis (mean±SD)**

| **Group** | | **n** | **TNF-α level in ankle (pg/mL)** | |
|---|---|---|---|---|
| | | | **Left ankle joint** | **Right ankle joint** |
| **Normal control group** | | 8 | 13.39±5.48** | 13.39±5.38* |
| **Model control group** | | 8 | 18.53±4.20 | 16.59±4.16 |
| **Compound I** | **Low dose group** | 8 | 10.16±5.82** | 10.71±4.61** |
| | **Medium dose group** | 8 | 9.57±5.10** | 9.63±3.02** |
| | **High dose group** | 8 | 8.66±4.33** | 8.98±7.44** |
| **Compound II** | **Low dose group** | 8 | 13.79±4.68** | 13.79±3.26** |
| | **Medium dose group** | 8 | 15.91±5.84 | 12.13±3.35** |
| | **High dose group** | 8 | 14.08±4.09** | 15.11±4.32 |
| **Compound III** | **Low dose group** | 8 | 12.93±4.15** | 14.13±4.20* |
| | **Medium dose group** | 8 | 13.62±3.42** | 12.02±5.91* |
| | **High dose group** | 8 | 11.05±3.96** | 13.85±3.38** |
| **Compound IV** | **Low dose group** | 8 | 13.56±4.22** | 11.62±4.18** |
| | **Medium dose group** | 8 | 12.59±4.69** | 15.51±4.10 |
| | **High** dose **group** | 8 | 14.65±4.26** | 13.68±4.78* |
| **Compound V** | **Low dose group** | 8 | 13.56±5.28** | 12.71±4.00** |
| | **Medium dose group** | 8 | 14.25±5.00** | 11.73±4.20** |
| | **High dose group** | 8 | 12.76±3.34** | 13.68±4.44* |
| **Compound VI** | **Low dose group** | 8 | 14.39±3.52** | 15.31±5.02* |
| | **Medium dose group** | 8 | 13.98±3.66** | 12.67±5.23* |
| | **High dose group** | 8 | 12.05±4.01** | 14.58±2.79** |

| | | | | |
|---|---|---|---|---|
| Note: compared with the model control group, *p<0.05, **p<0.01 | | | | |

### (5) Pathological examination results of hindfoot ankle joint of rats

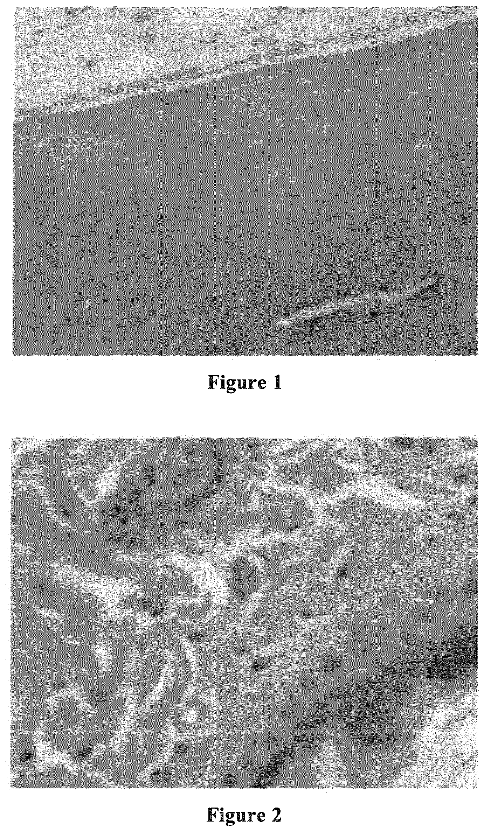

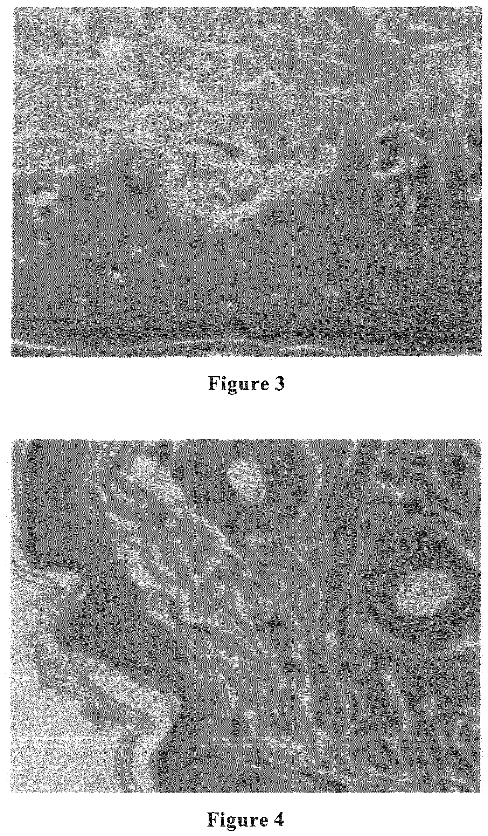

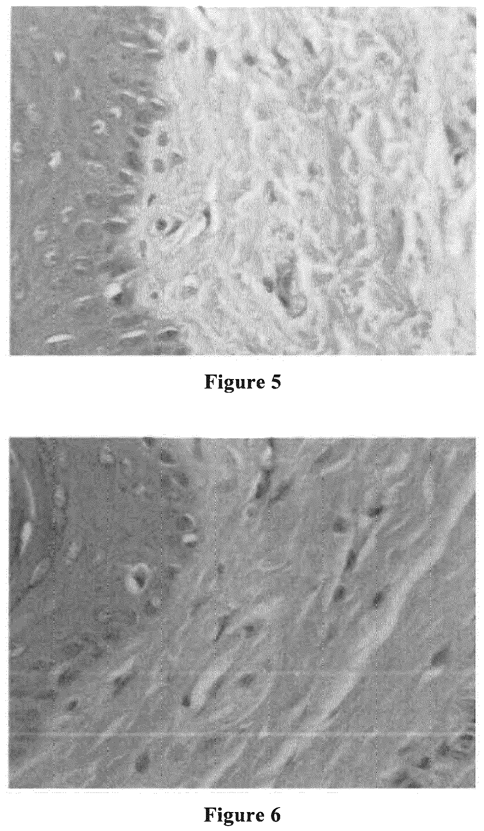

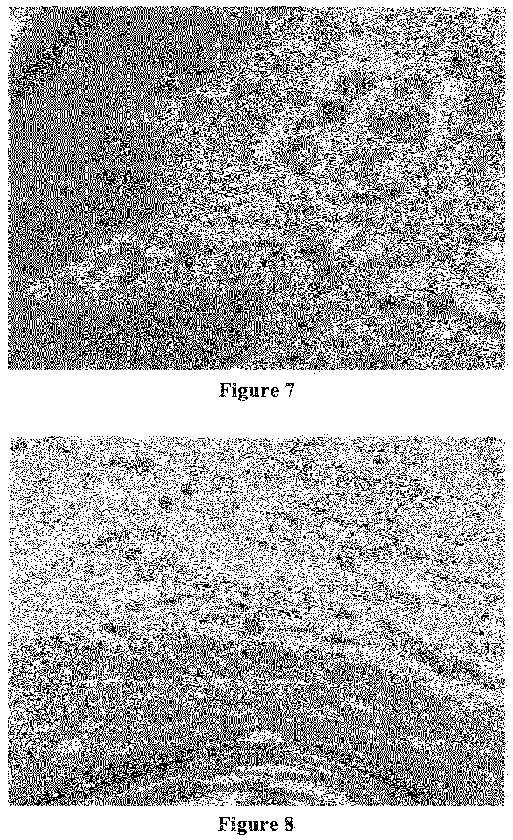

Pathological examination results of hindfoot ankle joint of rats were shown in table 6 and figure 1-7. Table 6 showed that, arthritis symptoms were obvious in the model control group with the most severe joint lesion. Therapeutic effects on arthritis were obvious in the high dose group, with significant difference compared with the model control group. Figure 1 showed that, there was no significant pathological change observed in joint bone, cartilage and synovial tissue of normal rats without inflammatory cell infiltration in the skin and subcutaneous tissue around bilateral joints. Figure 2 showed that, most joint synovial tissues of rats in the model control group were infiltrated by inflammatory cells, synovial cells degenerated with a minority of which proliferating, and subcutaneous tissue around joints had moderate or severe inflammatory response. Figure 3-7 showed that the lesion condition in the high dose groups of administration was lighter than that in the model control group.

**Table 6. Pathological examination score results of hindfoot ankle joint of rats (mean±SD)**

| **Group** | **Dose (mg/kg)** | **n** | **Joint lesion score** |
|---|---|---|---|
| **Normal control group** | 0.5mL | 4 | 0.50±1** |
| **Model control group** | 0.5mL | 4 | 4.50±0.58 |
| **Compound I** | 1.40 | 4 | 1.15±0.86** |
| **Compound II** | 1.80 | 4 | 2.50±2.65 |
| **Compound III** | 2.16 | 4 | 1.50±1.91* |
| **Compound IV** | 2.00 | 4 | 2.38±2.06 |
| **Compound V** | 1.80 | 4 | 3.75±2.06 |
| **Compound VI** | 1.48 | 4 | 1.88±1.26 |

| | | | |
|---|---|---|---|
| Note: compared with the model control group, *p<0.05, **p<0.01 | | | |

### (6) Conclusion

The present invention investigated the therapeutic effects of fat emulsion injection of Compounds I, II, III, IV, V and VI on chicken type II collagen and adjuvant co-induced arthritis in rats, and evaluated its pharmacological activity in the treatment of arthritis through the analysis of various indexes in rats with arthritis.

It can be seen from swelling degree of hindfoot of rat model and rats in administration groups and the results of treatment of hindfoot ankle joint that, certain effects occurred on decreasing the swelling degree of hindfoot and the thickness of hindfoot ankle joint of rats. Detection results of TNF-α in serum showed that it can lower TNF-α level in serum, and the results of pathological section of ankle joint of rats were substantially consistent with that of serology.

In conclusion, Compound I and its ester derivatives have therapeutic effects on this arthritis model, and it can be expected that the efficacy will be better with the increase of dose and the extension of administration time.

## Claims

1. A compound of Formula A for use in the treatment of arthritis, 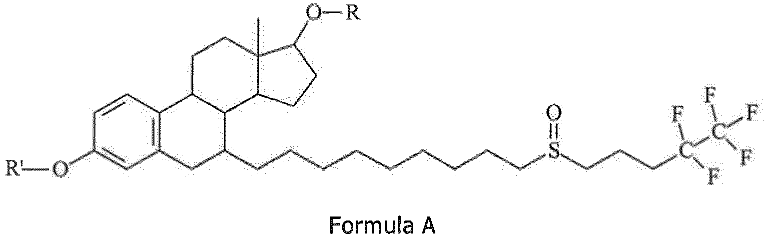 wherein,
substituent R' is selected from H, alkanoyl or alkenoyl having 2 to 4 carbon atoms,
substituent R is selected from H, alkanoyl or alkenoyl having 2 to 22 carbon atoms,
and when substituent R' is H, substituent R is linear or branched alkanoyl having 11 to 22 carbon atoms.

2. The compound for use of claim 1, **characterized in that**, said arthritis is rheumatic arthritis or rheumatoid arthritis.

3. The compound for use of claim 1 or 2, **characterized in that**, when R' is alkanoyl having 2 to 4 carbon atoms, said alkanoyl is acetyl.

4. The compound for use of any one of claims 1 to 3, **characterized in that**, said substituent R is alkenoyl having 11 to 22 carbon atoms.

5. The compound for use of claim 4, **characterized in that** substituent R contains one or more carbon-carbon double bonds.

6. The compound for use of claim 5, **characterized in that**, said linear or branched alkenoyl has 1 to 6, carbon-carbon double bonds and having 11 to 22 carbon atoms.

7. The compound for use of claim 6, **characterized in that**, substituent R is branched alkenoyl, wherein said double bonds can be in the main chain or in the branched chain.

8. The compound for use of claim 7, **characterized in that**, substituent R is undec-2-enoyl.

9. The compound for use of any one of claims 1 to 3, **characterized in that**, said substituent R is alkanoyl having 11 to 22 carbon atoms.

10. The compound for use of any one of claims 1-3 or 9, **characterized in that**, substituent R is selected from undecanoyl, docosanoyl, octadecanoyl and isostearoyl.

## Patentansprüche

1. Verbindung der Formel A zur Verwendung bei der Behandlung von Arthritis, 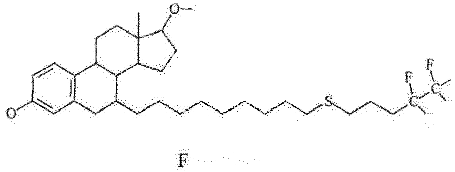 worin,
Substituent R' ausgewählt ist unter H, Alkanoyl oder Alkenoyl mit 2 bis 4 Kohlenstoffatomen;
Substituent R ausgewählt ist unter H, Alkanoyl oder Alkenoyl mit 2 bis 22 Kohlenstoffatomen;
und, wenn Substituent R' H ist, Substituent R lineares oder verzweigtes Alkanoyl mit 11 bis 22 Kohlenstoffatomen ist.

2. Verbindung zur Verwendung nach Anspruch 1, **dadurch gekennzeichnet, dass** die Arthritis rheumatische Arthritis oder rheumatoide Arthritis ist.

3. Verbindung zur Verwendung nach Anspruch 1 oder 2, **dadurch gekennzeichnet, dass** wenn R' Akanoyl mit 2 bis 4 Kohlenstoffatomen ist, das Alkanoyl Acetyl ist.

4. Verbindung zur Verwendung nach einem der Ansprüche 1 bis 3, **dadurch gekennzeichnet, dass** der Substituent R Alkenoyl mit 11 bis 22 Kohlenstoffatomen ist.

5. Verbindung zur Verwendung nach Anspruch 4, **dadurch gekennzeichnet, dass** der Substituent R ein oder mehrere Kohlenstoff-Kohlenstoff Doppelbindungen aufweist.

6. Verbindung zur Verwendung nach Anspruch 5, **dadurch gekennzeichnet, dass** das lineare oder verzweigte Alkenoyl 1 bis 6 Kohlenstoff-Kohlenstoff Doppelbindungen und 11 bis 22 Kohlenstoffatome aufweist.

7. Verbindung zur Verwendung nach Anspruch 6, **dadurch gekennzeichnet, dass** der Substituent R verzweigtes Alkenoyl ist, worin die Doppelbindungen in der Hauptkette oder der Seitenkette sein können.

8. Verbindung zur Verwendung nach Anspruch 7, **dadurch gekennzeichnet, dass** der Substituent R Undec-2-enoyl ist.

9. Verbindung zur Verwendung nach einem der Ansprüche 1 bis 3, **dadurch gekennzeichnet, dass** der Substituent R Alkanoyl mit 11 bis 22 Kohlenstoffatomen ist.

10. Verbindung zur Verwendung nach einem der Ansprüche 1-3 oder 9, **dadurch gekennzeichnet, dass** der Substituent R ausgewählt ist unter Undecanoyl, Docosanoyl, Octadecanoyl und Isostearoyl.

## Revendications

1. Composé de Formule A pour une utilisation dans le traitement de l'arthrite : 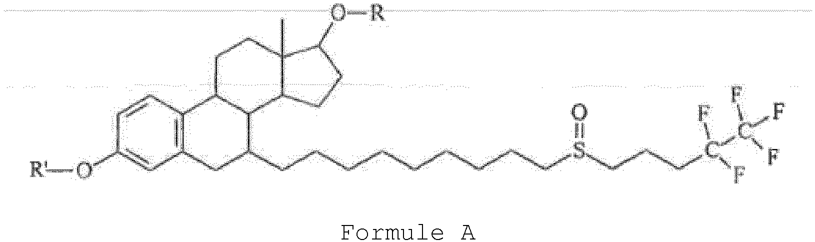 dans laquelle :
le substituant R' est choisi parmi H, alcanoyle ou alcénoyle ayant 2 à 4 atomes de carbone ;
le substituant R est choisi parmi H, alcanoyle ou alcénoyle ayant 2 à 22 atomes de carbone ;
et, lorsque le substituant R' est H, le substituant R est alcanoyle linéaire ou ramifié ayant 11 à 22 atomes de carbone.

2. Composé pour une utilisation selon la revendication 1, **caractérisé par le fait que** ladite arthrite est l'arthrite rhumatismale ou la polyarthrite rhumatoïde.

3. Composé pour une utilisation selon l'une des revendications 1 ou 2, **caractérisé par le fait que**, lorsque R' est alcanoyle ayant 2 à 4 atomes de carbone, ledit alcanoyle est acétyle.

4. Composé pour une utilisation selon l'une quelconque des revendications 1 à 3, **caractérisé par le fait que** ledit substituant R est alcénoyle ayant 11 à 22 atomes de carbone.

5. Composé pour une utilisation selon la revendication 4, **caractérisé par le fait que** le substituant R contient une ou plusieurs doubles liaisons carbone-carbone.

6. Composé pour une utilisation selon la revendication 5, **caractérisé par le fait que** ledit alcénoyle linéaire ou ramifié a 1 à 6 doubles liaisons carbone-carbone et a 11 à 22 atomes de carbone.

7. Composé pour une utilisation selon la revendication 6, **caractérisé par le fait que** le substituant R est un alcénoyle ramifié, lesdites doubles liaisons pouvant être dans la chaîne principale ou dans la chaîne ramifiée.

8. Composé pour une utilisation selon la revendication 7, **caractérisé par le fait que** le substituant R est undéc-2-énoyle.

9. Composé pour une utilisation selon l'une quelconque des revendications 1 à 3, **caractérisé par le fait que** ledit substituant R est alcanoyle ayant 11 à 22 atomes de carbone.

10. Composé pour une utilisation selon l'une quelconque des revendications 1 à 3 ou 9, **caractérisé par le fait que** le substituant R est choisi parmi undécanoyle, docosanoyle, octadécanoyle et isostéaroyle.
